Europäisches Patentamt

European Patent Office

Office européen des brevets

(11) Publication number: **0 595 005 A1**

# EUROPEAN PATENT APPLICATION

(21) Application number: 93114762.3

(22) Date of filing: **14.09.93**

(51) Int. Cl.5: **A61K 31/68**, //(A61K31/68, 31:505,31:44)

(30) Priority: **14.09.92 ZA 926990**

(43) Date of publication of application:
**04.05.94 Bulletin 94/18**

(84) Designated Contracting States:
**AT BE CH DE DK ES FR GB GR IT LI LU NL SE**

(71) Applicant: **VESTA MEDICINES (PROPRIETARY) LIMITED**
**Holpro House**
**1 Snell Street**
**Micor, Johannesburg 2092(ZA)**

(72) Inventor: **Serfontein, Willem Jacob**
**47 Selikats Village,**
**Selikats Causeway**
**Faerie Glen, Pretoria 0043(ZA)**

(74) Representative: **VOSSIUS & PARTNER**
**Postfach 86 07 67**
**D-81634 München (DE)**

(54) **Pharmaceutical preparations for lowering homocysteine levels, containing vitamin B6, folic acid and vitamin B12.**

(57) Pharmaceutical preparations for lowering blood and tissue levels of homocysteine are disclosed, comprising:
a) vitamin B6;
b) folate or a suitable active metabolite of folate or a substance which releases folate in vivo;
c) vitamin B12, with or without intrinsic factor
and optionally antoxidants, choline and/or betaine. a) and b) are provided in slow release form (2-8 hours) and c) is to be released immediately (within 20 minutes).

The present invention relates to pharmaceutical preparations for lowering levels of homocysteine or for the prophylaxis or treatment of elevated levels of homocysteine in patients and for counteracting the harmful effects associated with homocysteine.

Elevated homocysteine levels can be correlated with some of the principle causes of morbidity and mortality in the Western world, the so-called "Western" diseases, including such conditions as myocardial and cerebral infarction. Precocious vascular disease is the main single cause of death accounting for the majority of these deaths (New Eng J Med 1986, 314 : 488). It is generally agreed that nutritional factors play an important role in the etiology of this and the other Western diseases. The precise nature of the nutritional factors responsible for these diseases, is difficult to define but it can be stated with certainty that these are multi-factorial. Briefly, in the affluent Western societies, there is an overconsumption on the one hand of macro nutrients such as proteins, fats and refined carbohydrates, which are normally underconsumed in the Third World countries. Due to food refinement and all the other facets of food processing necessitated by increased urbanisation in the West, much of the micro-nutrients (vitamins, minerals) are lost. This results in a metabolic imbalance between macro-nutrients (especially proteins and fats) on the one hand and the essential micro-nutrients on the other hand which are necessary for the normal metabolism of the former. Under these conditions, abnormal metabolic pathways may be activated leading to the production of toxic and harmful intermediary products which in many cases are the cause of disease and which normally are not produced at all or only in very small quantities. The metabolism of the amino acid methionine is a good example, in which case excessive quantities of the toxic and unnatural amino acid homocysteine are produced.

Elevated homocysteine levels also occur in certain patients due to genetic causes and may also be caused by certain drugs, including certain vitamin B6 antagonistic drugs.

Normally, methionine is metabolised by the transmethylation and transsulfuration pathways to produce cysteine.

Three pathways exist by means of which blood and tissue levels of homocysteine are controlled to ensure homocysteine homeostasis:

1. Conversion into cysteine by means of the vitamin B6 dependent enzyme cystathionine $\beta$-synthase (CBS)
2. Remethylation to methionine which requires folate (as substrate) and vitamin B12 as co-factor.
3. Remethylation to methionine in which other methyl donors such as betaine participate.

Elaborate provision therefore exists in the healthy body to keep homocysteine levels in check. The reason for this is that homocysteine is a very toxic compound which in the chronic situation may affect a variety of systems and tissues in the body.

A pathological condition due to one or more of several hereditary enzyme defects wherein homocysteine levels are abnormally high, is known as homocysteinuria. This condition is often associated with high blood levels of homocysteine (often 200 $\mu$ mole/l or higher) and the associated clinical defects include the following:-

1. Disintegration of the vascular elastic interna due to binding of homocysteine to allysine residues of tropoelastin.
2. Inhibition of the process of polymerisation and cross linking in the formation of elastin and collagen.
3. Hyperplasia of arterial smooth muscle cells and synthesis of extracellular collective tissue.
4. Degradation of vascular glycocalyx and synthesis of extracellular connective tissue.
5. Pro-thrombotic effects (activation of Hagemann factor and stimulation of thromboxane 2 production by platelets).
6. Progressive premature artherosclerosis.
7. Accelerated osteoporosis (Metabolism 1985, 34 : 1073).
8. Precocious occlusive vascular disease frequently manifested clinically as myocardial infarction, stroke, pulmonary embolism (Am.J.Med.Sc. 1977, 273: 120) and peripheral vascular occlusion.
9. Abnormalities in eyes, skeletal system, central nervous and vascular systems.
10. Occlusive disease of cerebral, carotid and aorto-iliac vessels.
11. Occlusion or stenosis of renal arteries which often results in renovascular hypertension. (See for example: Metabolism 1985, 34 : 1073, Am J. Med. Sc. 1977, 273 : 120, Stroke 1984, 15 : 1014, Atherosclerosis 1988, 71 : 227.
12. The sex and age related variations in plasma homocysteine parallel well-established age and sex-related risk factors in atherosclerotic disease.

It has also been shown in many studies, that whereas lipid levels are not markedly different in coronary patients and controls, homocysteine levels are significantly different. (See for example J Am. Coll. Cardiol. 1990, 16:1114)

It is therefore now widely accepted that elevated plasma homocysteine is a risk factor independent of established risk factors such as cigarette smoking, hypertension and diabetes for generalised arteriosclerotic disease (Circulation 1989, 79 : 1180).

On the other hand, evidence exists which suggests that B6 deficiency independently of homocysteine may be associated with vascular disease stressing the prime importance of an adequate intracellular B6 status to prevent these diseases.

It is therefore now accepted in the art that elevated blood levels of homocysteine are highly undesirable. Normalisation of such elevated levels of homocysteine therefore constitutes a therapeutic goal as such without reference to any specific disease entity, possibly causally related to such elevated levels.

Evidence is mounting that high cholesterol levels alone are not the risk factor in astherosclerotic diseases as was previously believed. Before cholesterol contributes to vascular occlusion another form of damage occurs which is correlated with high homocysteine levels. Once that damage has occurred the beneficial effects of cholesterol-lowering drugs, in particular so-called statins become highly questionable, particularly when viewed in the light of side effects of such drugs (raising LPa, decreasing Q10, weakening the immune system, cataracts, GI disturbances, myositis, myocarditis). Nevertheless, the prejudice in favour of cholesterol depressants has been so strong that these adverse findings have, until now, been given inadequate coverage in the review literature.

The present invention is aimed at counteracting root causes of artherosclerotic disease which damage the blood vessels before cholesterol becomes a problem.

The clinical condition of homocysteinuria, is an inborn error of metabolism which is either caused by an enzyme defect in the transsulfuration pathway or a similar defect in the 5-methyl tetrahydrofolate dependent remethylation of homocysteine to methionine. Patients with this disease usually have very high fasting blood levels of homocysteine (in excess of 200 micromolar in homozygotes) and have a limited life expectancy due to early vascular complications. This rare condition must be clearly distinguished from other milder (but chronic) forms of homocysteinaemia which may arise from other causes - both external and internal - but which are clinically of much greater importance due to the vastly higher prevalence thereof. Accordingly, a need exists for reducing or preventing not only the extreme elevated homocysteine levels in cases of homocysteinuria, but also the much more moderately elevated homocysteine levels pertaining to homocysteineaemia.

Inadequate metabolic status individually of vitamin B6, folate and vitamin B12 have been recognised as determinants of heart and peripheral occlusive disease. At the same time, deficiencies (individually) of each of these vitamins have also been known to be associated with increased homocysteine levels. Thus vitamin B6 deficient humans have a 43 % reduction in cystathionine $\beta$-synthase (CBS) activity and they excrete increased quantities of homocysteine in the urine, reflecting the effect of an inadequate B6 status on homocysteine blood levels. A negative correlation exists between dietary B6 intake and blood levels of protein bound homocysteine.

Similar relationships have been described between B12 and folate levels individually on the one hand and blood levels of homocysteine on the other hand. These relationships have been described by several authors and have been summarised in the following publications:-

1. Stroke, 1984, 15 : 1012
2. Metabolism 1984, 34 : 1073
3. Metabolism 1988, 37 : 175
4. Scan J Clin Lab Invest 1988, 48 : 215
5. Atherosclerosis 1988, 71 : 227
6. Circulation 1990, 81 : 2004

The present state of the art knowledge on homocysteine and its involvement in disease is well summarised and presented in a recent review article (J.Lab.Clin Med. 1989, 114 : 473). In the course of own investigations into the relationship between B6, B12 and folate metabolic status, homocysteine metabolism and occlusive vascular disease, applicant has established that in addition to the known and published information on these relationships, certain other aspects - heretofore unknown or not appreciated or not correctly interpreted - are of prime importance in connection with treatment and prevention of homocysteine related occlusive vascular disease. In addition, by the judicious application of these findings, treatment of hyperhomocysteineaemia may be appreciably facilitated.

Regarding the treatment and prophylaxis of hyperhomocysteineaemia, it is known that vitamin B6, vitamin B12 and folate play a role in regulating the methionine - homocysteine pathway and controlling levels of homocysteine (David E L Wilken, Nicholas P P Dudman, Haemostasis 1989; 19 (supplement 1) : 14 - 23; Per Magne Ueland and Helga Refsum, J.Lab.Clin.Med. November 1989, 473 - 501. However, it was previously not recognised, that many patients develop hyperhomocysteineaemia not primarily because of a lack of the relevant vitamins, but often because of absorption problems, especially in the case of vitamin B12.

Accordingly there is a need for improvement in pharmaceutical compositions for lowering elevated homocysteine levels in plasma and counteracting adverse clinical conditions associated therewith, especially with respect to those patients in whom elevated plasma homocysteine levels are primarily related to absorption problems such as occur in many elderly patients. It is precisely in such patients that the problem of hyperhomocysteineaemia with accompanying vascular pathology is often a serious one.

In particular, there is a need to provide pharmaceutical compositions and dosage regimens which achieve adequate lowering of plasma homocysteine levels and counteracting adverse clinical conditions associated therewith in the greatest number of patients suffering from elevated plasma homocysteine levels covering substantially all age groups and preferably with relatively low dosages of active ingredients.

More particularly, there is a need for pharmaceutical compositions and dosage regimens which attain the aforegoing with surprisingly low dosage rates of folate as compared with the prior art.

In the present invention, special provision is made to overcome such problems. These preferably include the following galenical and biochemical variations:-

a) the use of pyridoxal instead of pyridoxine as a source of B6 activity;

b) the galenical presentation of the vitamins concerned in such a form that the rate of release of each vitamin is compatible with maximum absorption and utilisation;

c) the use of transdermal vitamin formulations which allows direct absorption through the skin of small quantities over prolonged periods. This is accomplished either through the use of appropriately formulated vitamin plasters or through the use of sub-lingual tablets.

Reference is made to applicant's copending patent application entitled "Compositions for the Treatment and Prophylaxis of Metabolic Disturbances in Infants", claiming priority of ZA-PA 92/6989.

Here pharmaceutical and dietary preparations are disclosed for the treatment or prophylaxis of elevated homocysteine and/or methionine levels in the blood of human infants and pathological disturbances connected therewith, said preparation comprising in combination:-

a) vitamin B 6 as such or in the form of a pharmaceutically acceptable acid salt, at least in part in the form of pyridoxal (PL) or a compound which in vivo readily releases PL without the intervention of oxidase enzyme or oxygen.

b) folate or a precursor of folate which releases folate in vivo, and

c) vitamin B12, with or without intrinsic factor, in the following ratios:-

a) . b) from 1:25 to 10 000 : 1

b) : c) from 1:1 to 50 000 : 1

The preparations are to be incorporated in infant bone feed mixes. That disclosure, by cross-reference, forms part of the present disclosure. The same applies to the contents of a study performed on behalf of the applicant and published after the priority date hereof in Am.J.Clinical Nutrition (1993), 57, pp 47-53.

In accordance with the invention there is provided the use in the manufacture of a pharmaceutical preparation for lowering levels of homocysteine or for the prophylaxis or treatment of elevated levels of homocysteine in a patient of a combination which comprises

a) vitamin B6;

b) folate or a suitable active metabolite of folate or a substance which releases folate in vivo;

c) vitamin B12, with or without intrinsic factor.

The invention is applicable to the lowering of total homocysteine blood levels if elevated by any known cause, including genetic causes (e.g. enzyme polymorphism) diets, drugs or depressed activity levels of folate, vitamin B6, vitamin B12 or any combination of these due to whatever cause, pregnancy, chronic renal failure, psoriasis, occlusive vascular disease, chronic liver disease, homocysteine-associated psychiatric problems. Drugs which induce elevated homocysteine levels include anticonvulsant drugs, xanthine bronchodilators (e.g. theophylline), methotrexate, nitrous oxide, and many others.

Preferably, in the preparation, the ingredients a) - c) are present in the following ratios by weight calculated on the basis of pure unphosphorylated pyridoxal (PL), pure vitamin B12 and pure folic acid:

a):b) from 100:1 to 1:10 and

b):c) from 100:1 to 1:50

The preferred ratios are:

a):b) from 50:1 to 1:1,5

b):c) from 15:1 to 1:2

more preferred ratios are:-

a):b) from 20:1 to 2,5:1

b):c) from 4:1 to 1:1

4

and in particular:-

a):b) from 20:1 to 5:1

b):c) from 2:1 to 1:2

The scope of the invention is intended to include a pharmaceutical preparation as such as aforesaid, wherein if the preparation is for oral use and any of the vitamin B6 is represented by pyridoxine (PN), such PN is formulated in slow-release form. This is particularly advantageous in the context of PN, because of the limited capacity of the liver to convert PN into PLP and the resultant risks of excess PN in plasma leading to poor utilisation and undesirable entrance of PN into peripheral cells and erythrocytes.

Pharmaceutical preparations containing the aforesaid active ingredients have been described, albeit for totally different purposes and mostly in ratios differing from the aforesaid ratios or at least from the preferred or more preferred ratios. In GB-PS 1201 014 (examples 6 and 7) the ratio of a):b) = 3:1 and that of b):c) = 1000:5. No indication is disclosed for these dragees. GB-PS 2254 556, published after the priority date of the present disclosure, also discloses compositions, only some of which contain in combination folic acid, vitamin B12, vitamin B6. No distinction is drawn between pyridoxine, pyridoxal and pyridoxamine. These compositions are intended for adolescent girls. GB-PS 149 3993 discloses compositions for treating obesity. Pyridoxal is not disclosed. GB-PS 2145 331 discloses all these ingredients but in quite different ratios and in dosages which are partly too high and partly too low for the purposes of the present invention. GB-PS 2197 587 describes a "blood conditioning tonic" for race horses. Pyridoxal is not disclosed. GB-PS 1431 841 discloses preparations for cataract treatment. The ratios are different from those according to the invention and pyridoxal is not disclosed. GB 101 3939 discloses compositions for paediatric purposes in ratios which overlap the broadest ratio according to the invention, but the important feature that the vitamin B6 must be in the form of pyridoxal or suitable precursor thereof is not disclosed. This also applies to EP-0144051, EP 0121 036 or PCT WO 83/00085.

Not one of the aforesaid references discloses such combinations for the treatment or prophylaxis of elevated homocysteine levels in plasma, or the crucial role of pyridoxal in that context, which is the only useful form which when it enters peripheral cells or erythrocytes is directly converted there into active pyridoxal phosphate (PLP).

It is now realised that such intracellular PLP is the sole form in which vitamin B6 controls homocysteine levels in plasma.

The preparations in accordance with the invention are formulated to provide approximate daily dosages as follows ($\mu$g/d/kg body weight).

|  | a) Vitamin B6 | b) Folic Acid | c) Vitamin B12 |
|---|---|---|---|
| Broadest range | 15-750 | 1,5-150 | 1,5-75 |
| preferred range | 30-400 | 7,5-50 | 3-15 |
| more preferred range | 75-250 | 10-30 | 7-10 |
| typical example | 150 | 15 | 7,5 |

These dosages may be exceeded somewhat for short durations, e.g. at the beginning of the treatment. Also, where the daily dosages are divided into several dosage units to be administered at different times of the day, the compositions may differ to provide optimum effect in accordance with circadien variations in homocysteine production. The latter may fluctuate in a manner depending on time, on meal intake, its quantity and composition. The dosage regimen may be programmed to be optionally adapted to a predetermined daily dietary programme.

Preferably the preparation is formulated to make available to the patient the vitamin B6 and preferably also the folate over a period of more than 1 hour and to make available an effective dosage of the vitamin B12 in less than 1 hour after administration. This feature is considered to contribute materially to the efficacy of the invention and is considered to be novel and inventive per se.

The preparation may be galenically formulated for parenteral administration, preferably by infusion or by intramuscular injection. The latter form inherently provides for a retarded availability of the ingredients, which effect may be further enhanced by depot forms of formulation.

Preferably the preparation combines all three essential ingredients in a single dosage form, which except for very drastic cases of elevated homocysteine levels is preferably designed for oral administration.

However, it is possible within the scope of the invention, to provide separate ingredients of the preparation in separate distinctive dosage forms, e.g. capsules, tablets or coated tablets, preferably combined in a single package e.g. a blister pack or similarly ordered package, designed to facilitate or prescribe to the user the combined administration of the dosage units according to a specific dosage regimen. Such dosage regimen may optionally be time programmed, providing for different dosage rates during different periods of a course of treatment. Packages designed for that purpose are known per se and require no description.

Preferably at least the vitamin B6 should be galenically formulated for slow release of the compound over a period of not less than 2 hours. Likewise the folate or precursor thereof is preferably so formulated.

On the other hand, it is preferred for the vitamin B12 (with or without intrinsic factor) to be galenically formulated for the preparation to release an effective dosage, preferably at least 90% of the vitamin B12 (with or without intrinsic factor) to the patient, more particularly the stomach in less than 1 hour after administration.

The vitamin B6 as such or in the form of pharmaceutically acceptable acid addition salt may be in the form of pyridoxine (PN) or its phosphate (PNP). However, for the reasons already stated above, it is preferred for the vitamin B6 to be represented at least in part by pyridoxal (PL) or a compound which readily releases PL in vivo without the intervention of oxidase or oxygen, because this avoids situations where the normal PN - PL metabolic pathway may be compromised, as may happen e.g. due to genetic or pathological or drug-induced conditions.

Nevertheless, because most patients, in particular non-infants have a reasonable capacity for utilising PN it can be advantageous to employ a mixture of PN and PL in the following ratio:-

PL:PN =            from 1:10 to 10:1
preferably         from 1:6 to 4:1
more preferably    from 1:6 to 1:1
e.g. 1:4

Likewise it is preferred for PL or its precursor to be provided in a non-phosphorylated form, to avoid situations where the dephosphorylation step may be compromised. It is pointed out that only PL is capable of passing from the plasma through the cellular membranes into most cells where it is subsequently converted into pyridoxal phosphate (PLP), the active intracellular form of PLP. Also as will be explained elsewhere herein, PL itself plays a very active role in certain physiologically important reactions relevant to the present invention. For these reasons PL itself is a preferred form of vitamin B6 in the context of the present invention.

Vitamin B 12 may be used in the form of cyanocobalamin or hydroxycabalamin or both.

"Intrinsic factor" in this art, in the context of vitamin B12 denotes substances (which are for example in nature released by the gastric mucosa of the stomach when functioning normally) with which vitamin B12 forms complexes to facilitate absorption.

Advantageously the vitamin B6 is galenically formulated to be released over a period of 2 to 8 hours, whereas the vitamin B12 (with or without intrinsic factor) is formulated to be released in less than $\frac{1}{2}$ hour. More particularly the vitamin B6 is galenically formulated to be released over a period of 2 to 8 hours, preferably 3 to 6 hours, more preferably 4 to 6 hours and the B12 over a period of 5 - 30 minutes.

Preferably, the folate as well is galenically formulated to be released by the composition in not less than 2 hours, preferably 2 to 8 hours, more preferably 3 to 6 hours, e.g. 4 to 6 hours.

The preferred compositions contain vitamin B6 and, preferably folate in a part of the composition adapted as a slow, timed release composition and containing the vitamin B12 (with or without intrinsic factor) in another part adapted for fast release. Examples of such compositions include the following:

a) a bi-layered tablet,

b) a coated tablet, containing the vitamin B12 in a rapidly dissolving coating; or

c) a pharmaceutical composition in granular form, loose or in a capsule.

Novelty and inventiveness is claimed to reside in the feature as such of combining folate and vitamin B12 in a combination, wherein the former is galenically formulated or adapted to be administered in a slow, timed release manner and the latter is formulated or adapted for fast release. This feature is considered as a further aspect of the present invention, to be applied as such or in combination with the remaining features of the invention herein disclosed.

The manner of putting that aspect of the invention into effect is as disclosed herein in conjunction with the preceding aspects of the invention.

Furthermore, apart from the proven toxicity of homocysteine, it has in addition now been found that elevated homocysteine levels in plasma are also indicative of free radical activity and of a general vitamin deficiency, and notably a deficiency of those vitamins which control free radicals in plasma. Free radicals in plasma as such, are a risk factory, which can be associated with serious diseases, notably vascular diseases. Accordingly, the invention preferably provides for the co-administration with the aforesaid substances a) or b) or c) of an antioxidant, more particularly d) vitamin C (ascorbic acid or salt thereof) and/or e) vitamin E (more particularly in the form of d-α-tocopherol acetate), and/or g) selenium, preferably as selenised yeast, and/or h) coenzyme Q10, preferably two or more of these together.

Components d), e), g) and h) are preferably incorporated in the same pharmaceutical preparation as a) and/or b) and preferably likewise in a slow-release form.

The use of antioxidant vitamins in combination with folate, more particularly in slow-release form (and preferably in combination with vitamin B6 and vitamin B12) for the purpose of counteracting the adverse clinical effects associated with elevated homocystein levels, notably vascular disease is considered novel and inventive per se.

The daily dosage rate in the context of the invention for vitamin C is preferably from 100 to 1200, more preferably 200 to 700, in particular about 500 mg/70kg, and that for vitamin E is preferably 80 to 1000, more preferably 150 to 600 in particular about 400 mg/70kg. Another antioxidant which can be used, preferably in addition to either or both of d) and e), is f) β-carotene, at daily dosage rates of 1 to 20, preferably 5-15, e.g. about 7,0 mg/70kg. Selenium is used at a dosage rate of 20-400 $\mu$g, preferably 100-300 $\mu$g, e.g. about 200 $\mu$g/70kg and coenzyme Q at a dosage rate of 10-100 mg, preferably 25-30 mg, say 20 mg/70 kg.

The ratios of the antioxidants to other ingredients may be:

b) to:d) = 1:2000 -1:50, preferably 1:1000 - 1:100, e.g. 1:500
b) to:e) = 1:1800 - 1:40, preferably 1:900 - 1:90 e.g. 1:400
b) to:f) = 1:0,5 - 1:30, preferably 1:2 - 1:15, e.g. 1:7,5
b) to:g) = 1:0,4 - 1:0,02 preferably 1:50 - 1:15 e.g. 1:20
b) to:h) = 1:100 - 1:5 preferably 1:50 - 1:15 e.g. 1:20.

The scope of the invention also extends to compositions or preparations as aforesaid comprising optionally choline or betaine or both, and these are preferably formulated in slow release form. Choline is a precursor of betaine. These substances are incorporated to provide a daily dosage rate of 0,007 - 0,1, preferably 0,01 - 0,05, more preferably 0,014 - 0,03 g/d/kg body weight, e.g. a daily adult dosage rate of 2,0 g betaine. HC*l* combined with 10 mg PN.HC*l*. The use and advantages of betaine and choline in slow-release form are inventive per se.

By employing betaine or choline in slow release form it is possible to reduce the dose substantially for a given homocysteine-reducing effect. In this manner excessive levels of methionine are also counteracted. At the same time the co-administration of vitamin B6, preferably PL counteracts excessive fluctuations of blood amino acid levels. The release characteristics may be similar to those disclosed above for vitamin B6 and folate, preferably such that 90% are released in 4-6 hrs.

Betaine and/or choline can even substantially replace either or both of vitamin B12 and folate, because betaine, like vitamin B12 and folate, promotes the methylation of homocysteine.

According to another aspect of the invention, provision is made for the application of the active ingredients (vitamins) concerned (one or more) by the use of appropriately formulated:-

1) sub-lingual tablets (especially in the case of coenzyme Q),
2) plasters designed for skin absorption,
3) rectal pesaries,
4) suitably formulated gels or ointments, or
5) suitably formulated and concentrated solutions (aqueous, non-aqueous) of vitamins applied to the skin and/or other suitable tissues.

Generally, such preparations are prepared for the direct absorption of one or more of these vitamins through various tissues and membranes including the skin, nasal membranes, sub-lingual membranes, rectal membranes.

Preferably also such preparations may contain one or more permeation enhancers such as the mono-esters of glycerol which are known for that purpose in the art.

The principle advantage of such parenteral formulations (applying the term "parenteral" in a broad sense) is the fact that the inconvenient, unpleasant and often costly application by means of injections can be avoided. This is of special significance in the case of vitamin B12 and coenzyme Q10.

It has also been found necessary to use suitable vehicles which are adapted to facilitate and/or control the release of the vitamins for absorption through the tissues concerned. Such preparations may include the use of certain gels or suitably formulated tablets. Since the three vitamins concerned are normally not absorbed at the same rate, in the various systems concerned, it has been found necessary to vary the relative concentrations of the vitamins in such preparations to allow for even, parallel and protracted absorption of the vitamins. Alternatively, and preferably, a composite plaster containing 3 zones, each loaded with one of the relevant vitamins and each containing its own permeation enhancers may be prepared. Because in the case of folate and B6, absorption problems are not so serious as those of B12 (especially in the aged), this form of parenteral administration is frequently resorted to only in the case of B12 and coenzyme Q10.

According to yet another aspect of the invention, absorption problems (especially with respect to B12 absorption, e.g. in the elderly) are overcome by using the three vitamins in substantially differing concentration ratios in such a manner that the folate and B12 components are presented in higher quantities relative to the B6 component (e.g. pyridoxal). The principle is illustrated in the following table. The dosage forms in accordance with the invention are to be formulated accordingly:-

## Table

## Concentration ranges of pyridoxal, folate and vitamin B12 in pharmaceutical formulations

The following quantities refer to one <u>daily dose</u> for an adult patient of approximately 70kg body weight. (PL=pyridoxal; Fol=folate; B12=Vitamin B12) Quantities are given in milligrams per day.

| Formulation type | PL | | Folate | | B12 | |
|---|---|---|---|---|---|---|
| | Range mg | Preferred mg | Range mg | Preferred mg | Range mg | Preferred mg |
| Normal (no absorption problem) | 2-5 | 5 | 0,2-15 | 1,0 | 0.1-2 | 0.5 |
| Special (to overcome absorption problems) | 2-50 | 5 | 2-15 | 5 | 0.2-5 | 1,0 |

One of the mechanisms by which homocysteine causes vascular and other organ pathology is by means of oxidative modification of lipoproteins. It is known that homocysteine potentiates the oxidation of lipoprotein cholesterol with the formation of oxysterols (Bioch. Biogh. Acta 1987, <u>917</u>:337) and it is also known that oxycholesterol is much more atherogenic than cholesterol itself.

According to yet another aspect of the invention, provision is made to suppress the homocysteine catalyzed oxidation of lipoprotein cholesterol. This may be of benefit to patients with very high homocysteine levels (perhaps due to genetic abnormalities, or for other reasons) in whom, during treatment, homocysteine levels decline slowly to normality over a long period (e.g. weeks). Homocysteine-induced oxidation of cholesterol can be suppressed by means of antioxidants (e.g. $\beta$-carotene, vitamin E, vitamin C,coenzyme Q, etc.). In this respect, it has surprisingly been found that pyridoxal (PL) itself has anti-oxidant (anti-free radical) activity. Thus when used as provided for in the invention, PL serves a variety of purposes as outlined above, including that of an anti-oxidant.

However, particularly in severe cases of homocysteinuria (e.g. due to genetic disorder) it is advantageous to include one or more powerful anti-oxidants drawn from the list of compounds mentioned above.

In such cases it may also be necessary to administer choline or betaine as herein provided for. Thus, according to yet another aspect of the invention, a pharmaceutical formulation comprising vitamin B6 (preferably at least in part in the form of pyridoxal) folic acid and vitamin B12 in combination with one or more anti-oxidants is provided for as illustrated in the following table:-

| Compound | Range (mg) | Preferred (mg) | For Example (mg) |
|---|---|---|---|
| B6, preferably as Pyridoxal | 2-50 | 5-15 | 5,0 |
| Folate | 0,2-15 | 0,5-3 | 1,0 |
| Vitamin B12 | 0,2-5 | 0,5-1,5 | 0,5 |
| Anti-oxidants | | | |
| ß-carotene | 1-12 | 5-15 | 7,0 |
| d-$\alpha$-tocopherol acetate | 10-1000 | 50-700 | 500 |
| Ascorbic acid | 30-1000 | 100-700 | 500 |
| Coenzyme Q10 | 10-100 | 15-50 | 20 |

In different formulations, one or more of the anti-oxidants are preferably included with the first three compounds.

All such formulations are preferably formulated in such a manner that both a rapid release phase and a retarded release phase is present as previously outlined. The anti-oxidant component(s) may be present in either phase.

The pharmaceutical compositions are not only to be used in the treatment of raised homocysteine levels induced nutritionally, genetically or as a result of a variety of diseases, but also in those cases where the elevated homocysteine levels are drug induced or in combination with a B6 or folate antagonistic drug, which has a tendency to raise homocysteine levels. Examples of other situations in which blood homocysteine levels may be elevated are the following: post-menopausal women, liver failure, leukemia, other cancers, chronic renal failure. Slow-release formulation of PL prevents excessive liver oxidation to the biologically inactive pyridoxic acid.

Of great importance to the inventive concept behind the pharmaceutical compositions in accordance with the invention is inter alia the appreciation of the importance of making available at the correct rate and time vitamin B6 in the form of its vitamer pyridoxal which compound applicant has also surprisingly found to be readily absorbed through tissues other than those in the gastro-intestinal tract. The normal source of vitamin B6 in the pharmaceutical industry is pyridoxine, usually in the form of its hydrochloride. However, if pyridoxine is used according to the present invention, it is preferred that it should be administered in a slow release form so designed that the pyridoxine is made available to the plasma sufficiently slowly to prevent overloading of the body and permit the rapid and efficient transformation of the PN into PL and hence into intracellular pyridoxal phosphate (PLP). However, for reasons which will become apparent from what is described further below, it is preferred to employ at least in part pyridoxal as such as the source of vitamin B6 or alternatively, a compound, which in vivo rapidly releases pyridoxal without the intervention of oxidase or oxygen. Further details of suitable sources of pyridoxal are described in European patent application no 90 100834.2 and corresponding applications in other countries, which by cross-reference thereto are to be considered as part of the present disclosure. A further advantage of pyridoxal over pyridoxine is that the former does not require the liver enzyme systems necessary for the activation of pyridoxine. It therefore has clear advantages as a B6 source in non-parenteral administration forms as herein provided for. Applicant has surprisingly found, that small quantities of pyridoxal in non-parenteral formulations are sufficient to provide adequate blood levels when used over long periods of time. This in turn has considerable advantages from the galenic point of view in the formulation of such preparations.

It is an important preferred feature of the invention that the vitamin B6, even if in the form of pyridoxal or one of its precursors and also folate, if present, should be formulated as a slow, timed release composition, whereas the vitamin B12 should be adapted for immediate release, especially in parenteral formulations.

The inventive concept is inter alia based on research leading to an improved understanding of how fluctuations in cystathionine synthase activity represent a metabolic adaptation and control mechanism of homocysteine blood levels in response to methionine overload. An important part of this concept is the realisation of the importance of administering vitamin B6 in the correct manner and at such a rate that plasma pyridoxal (PL) as distinct from plasma pyridoxal phosphate (PLP) levels are optimally maintained. The conventional parameter for monitoring the vitamin B6 status is to determine plasma PLP. However, it has surprisingly been found that no clear and reliable correlation is possible between plasma PLP and plasma PL, the ratio of which can vary between very wide limits. It was found surprisingly that in the context of controlling homocysteine blood levels one of the key factors is plasma PL (and not PLP). The plasma PL dependent conversion of homocysteine into cystathionine is of particular importance in the day to day control of blood homocysteine levels. This reaction is rapidly activated by administering pyridoxal (PL) since PL is rapidly available intracellularly for the formation of the active co-enzyme pyridoxal phosphate (PLP). Another control mechanism utilised by the body for the maintenance of low blood homocysteine levels is increased transaminations of methionine away from the transsulfuration pathway. This transamination pathway is not important in the normal healthy individual with normal blood homocysteine levels but is activated when blood homocysteine levels rise. This pathway is also B6 dependent and again plasma PL is an efficient activator of this pathway. Applicant has demonstrated the dependence of transamination enzymes (ALT, AST) on intracellular B6 activity. Thus in the overall metabolic control of blood homocysteine levels, plasma PL plays an important role since it is involved in different reactions at different sites and this effect can be clinically best utilised by administering B6 in the form of PL especially in the case of parenteral formulations. This was not previously appreciated, nor was the important role of PL specifically appreciated for the stimulation of transamination reactions in general realised. PL as such (as distinct from PLP) acts as a co-enzyme for transamination of amino acids.

An additional bonus resulting from supplementing plasma PL levels is the beneficial effect thereof on raised and distorted blood lipid patterns which greatly enhance the atherogenesis induced and accelerated by even mildly elevated blood homocysteine levels. A synergistic effect results from controlling blood lipid and homocysteine levels simultaneously through the agency of plasma PL.

The administration of PL instead of PN also effectively deals with the problem that approximately 20 - 25% of all patients suffer from a depressed ability to utilise PN due to genetically induced enzymatic polymorphism.

Apart from its interaction with homocysteine via lipid metabolism in this regard, vitamin B6 is desirable because it also counteracts the damaging effects of homocysteine to the vascular wall due to its involvement as a co-factor for the enzyme which catalyses cross-linking in the vascular structural proteins (lysyl oxidase). In this respect both PL and intracellular PLP are directly involved. Thus the ultimate clinical advantages of vitamin B6 in the form of PL go beyond control of blood homocysteine homeostasis.

Even better effects on lipid metabolism are obtained when PL is used in a slow release or timed release pharmaceutical formulation and/or when used in parenteral formulations. Applicant has surprisingly found that after bolus oral doses of PL, much of the administered dose is oxidised to inactive pyridoxic acid in the liver but this is not the case when PL is given in a timed release formulation or when PL is given in parenteral or transdermal formulations as herein described. Thus, the efficacy of PL as a drug and for the purposes of the present invention is greatly increased by administering it as indicated above. In addition, small quantities of PL are absorbed twice as fast as pyridoxine by both gastro-intestinal tissues as well as other tissues. The problem of liver oxidation of PL can be further circumvented by selecting a route of administration which minimises this problem. Applicant has surprisingly found that PL is readily absorbed transdermally as well as sub-linqually if the vitamin is formulated in the right vehicle. Such formulations also have considerable advantages in the case of both vitamin B12 and folate. In the case of vitamin B12, it is well known that the requirement of intrinsic factor for adequate absorption after oral administration, frequently causes absorption problems, especially in elderly patients. Applicant has surprisingly found that small quantities of vitamin B12 are readily absorbed transdermally as well as sublinqually. In the latter case, a rapidly dissolving tablet was found to form a suitable and rapidly absorbed depot under the tongue. Small quantities of vitamin B12 are also readily absorbed transdermally from a variety of vehicles. It was also found possible to produce both sub-linqual and transdermal formulations from which adequate absorption of folate takes place. In all these parenteral formulations, the vitamins are slowly absorbed. For this reason, the advantages of such parenteral formulations are only realised when they are used over long periods of time.

Furthermore, applicant has surprisingly found that for purposes of controlling blood homocysteine levels, the combination in accordance with the invention of PL, folate and vitamin B12 produces advantageous effects which go substantially beyond what might be expected from a simple additive effect of the action of these drugs. Thus, an unexpected synergism exists when vitamin B12, folate and PL are given concurrently and this effect can be even greater when the vitamins are given in conjunction with a biological methyl donor such as choline or betaine. This synergism is evidenced by:

1. Better control of blood homocysteine levels at lower dosage levels of each.
2. A tendency to restore to normality distorted blood amino acid patterns which are sometimes seen when betaine is given alone.
3. In the presence of both folate and PL, methionine levels do not rise as much after betaine due to activation of alternative metabolic pathways.
4. The presence of PL limits damage to structural proteins, especially in the vascular bed.
5. Clinical tests. (See examples)

This synergism may further be appreciated from the fact that PL stimulates a process which ultimately leads to the reduction of the methionine pool (through conversion of homocysteine into cysteine) whereas both vitamin B12 and folate stimulate processes which do not lead to a reduction of the body's methionine pool but mere recycling. The resultant methionine remains available for reconversion into homocysteine. PL (in its own right and distinct from PLP) has co-enzyme activity for the enzyme cystathionine synthase. Cystathionine synthase activity can be stimulated in a dose dependent manner by intracellular PLP and PL, both of which increase after administration of PL.

Folate increases the demand for intracellular PLP and therefore for extracellular PL which is the immediate source and precursor of intracellular PLP. This further indicates the necessity of administering PL simultaneously with the folate and preferably at proportionate rates.

According to one aspect of the invention, a sub-linqual tablet (preferably suitably buffered) is produced in such a manner that the PL, vitamin B12 and folate components are liberated and absorbed mainly under the tongue. Such a tablet can also be formulated to contain all or any one of the three vitamins for use where patient problems are related to only one of these vitamins. A typical example would be the treatment of raised homocysteine blood levels and/or psychiatric problems with or without anaemia in the elderly arising from a chronic B12 deficiency. (New Engl. J. Med. 1988, 318:1720). The use of such a sub-linqual B12 tablet, is particularly effective and useful in the elderly with a deficiency of intrinsic factor since the use of such a tablet obviates the use of repeated vitamin B12 injections. Sublingual tablets of Q10 are also an effective vehicle for administering coenzyme Q10 for the purposes of the present invention.

According to another aspect of the invention, a plaster containing PL, vitamin B12 and folate in a suitable carrier for transdermal absorption is produced. The rate of transdermal absorption from such a depot, can be further controlled by the application of suitable permeation enhancers and suitable membranes which control the rate of diffusion of the vitamins. Again such a plaster may contain all or any single one of the three vitamins and it would have the same indications for the treatment of vitamin B12-deficient elderly patients as in the example above. However, since the conditions for absorption are different for the three vitamins, this form of application is preferred when only one vitamin at a time is to be administered, e.g. vitamin B12 for elderly vitamin B12-deficient patients.

Applicant has found that PL is more readily absorbed from such parenteral depots than the other two vitamins. This method of application has other distinct advantages. In contrast to PN, PL does not require hepatic activation and the PL entering the circulation from parenteral depots, is directly available for metabolic use inside cells. Because hepatic oxidation of PL to inactive pyridoxic acid is largely prevented in this manner, it is possible to use smaller doses of PL, with corresponding cost advantages.

According to another aspect of the invention, a formulation containing vitamin B6 (preferably in the form of pyridoxal), folate and vitamin B12 is produced in tablet form for oral use, preferably in such a manner that the vitamin B12 component is liberated immediately in the stomach (preferably within 20 min.) while the other two components are contained in a timed release matrix in the tablet in such a manner that the total dose of PL and folate is liberated over a period of 2 - 8 hours, preferably 3 - 6 hours, for example 3 - 4 hours.

According to another aspect of the invention a bi-layered tablet is produced in such a manner that one layer of the tablet containing the B12 component is rapidly dissolved in the stomach while the other layer of the tablet consists of a timed release matrix in such a manner that the PL and folate components are liberated over a period of 3 - 4 hours as stated above. Alternatively, a tablet may be formulated which is coated on the outside with a rapidly dissolving B12 containing layer, such coating covering an inner layer consisting of a timed release matrix containing PL and folate as before.

In yet another variation of the invention an effervescent tablet is composed consisting of betaine, PL, folate and vitamin B12. Again such a tablet may be bi-layered, one rapidly dissolving layer containing the vitamin B12 component, while the other three components are present in timed release granules, incorporated into the second layer. Alternatively the latter may be present in the core of such a tablet which contains the vitamin B12 component in an outer, rapidly dissolving coating.

In yet another variation of the invention a powdered mixture (preferably in granulated form) is provided, consisting of one or more or all of the following ingredients:

1) Vitamin B12 in the form of rapidly dissolving granules
2) Choline or betaine in the form of slowly dissolving granules
3) PL in the form of slowly disintegrating granules in such a manner that the total dose is liberated in 4 - 6 h.
4) Folate, also in the form of slowly disintegrating granules.
5) One or more antioxidants.

For the optimum use of the invention it is advisable to monitor the homocysteine levels (total) in human plasma. A method developed by the applicant's research team, suitable for that purpose is described in JB Ubbink, et al J. of Chromatography (1991), 565, 441-446.

The general pharmacologies and toxicologies of the individual ingredients of the pharmaceutical preparations in accordance with the invention are well documented in the art and require no further description. The dosage rates in accordance with the invention are below accepted toxicity limits. It is also generally accepted that the administration of folate is contraindicated in the event of a depressed vitamin B12 status except if vitamin B12 is effectively supplemented at the same time.

The clinical tests hereinafter described reflect in summary the outcome of extensive clinical experiments. In these trials the following procedures were followed:-

a) After thorough analysis and statistical evaluation of total blood homocysteine concentrations in 349 adult caucasian men the cut-off limit between the "normal" homocysteine range and elevated homocysteine levels was set at 16.3 $\mu$mol/l. That cut-off limit has been employed for all other evaluations herein described.

b) All homocysteine determinations were performed by the method of JB Ubbink, et al; J. of Chromatography (1991), 565, 441-446.

c) In comparative trials all patients were ignored who at the commencement day of the trial already showed homocysteine levels below the abovementioned cut-off level.

## Example 1: Results of comparative trials

Comparative trials were conducted to compare the efficacy of preparations in accordance with the invention with comparable preparations but containing only a single active ingredient and with placebo.

The tablets containing only folate (0,65 mg) respectively only vitamin B6 (2,5 mg PL + 7,5 mg PN) only were formulated as slow release tablets as described in Example 3.

The tablets containing vitamin B12 (20 $\mu$g cyanocobalamine) only were formulated as immediate release tablets in the manner described in the same example.

The compositions in accordance with the invention tested in this trial had the following compositions:-
Invention "A": exactly as described in Example 2.
Invention "B": prepared as described in Example 3, however, with the following changes in composition:-
cyanocobalamin 400 μg, folic acid 1 mg.

The dosage regimen was 1 tablet/capsule daily, taken in evenings after meals for 42 days, except in the case of Invention "B", where the trial was continued for 56 days.

The results are summarised in the following table A:-

| Composition | Number of Patients | Homocysteine levels (μ mole/*l* | | | | Successful Treatment | |
| | | Starting | | 42 days | | | |
| | | Mean | SD**) | mean | SD**) | number | percentage |
| Placebo | 18 | 28,14 | 22,41 | 28,14 | 22,34 | 1 | 5,5 |
| B12 | 16 | 26,19 | 16,17 | 23,56 | 14,88 | 5 | 31,3 |
| folate | 16 | 29,02 | 15,06 | 16,89 | 7,85 | 10 | 62,5 |
| B6 | 16 | 27,26 | 14,95 | 24,21 | 12,40 | 3 | 18,8 |
| Invention "A" | 18 | 33,63 | 20,64 | 14,88*) | 6,34 | 12*) | 66,7 |
| Invention "B" | 12 | 28,57 | 14,64 | 11.46*) | 3,09 | 11*) | 91,7 |

*) see further comments
**) standard deviation

In addition to the results apparent from Table "A" the following important observations and comments apply:-

A) For "successful treatments" only those patients were counted whose homocysteine levels actually dropped below the cut-off level of 16,3 μmol/l. Cases where the levels had indeed dropped, but not below the cut-off level, were ignored. These cases did, however, have a positive effect on the final (42 days) mean values reflected in the table.

B) In the case of successful "single-substance" treatments no marginal cases were observed, for which one might have expected a further substantial improvement after prolonged treatment, beyond 42 days.

C) In the case of Invention "A" (containing a relatively low concentration of active B12 and no pyridoxal) the rate of improvement in some patients was slightly slower than in the case of Invention "B". At least 3 patients had shown such clear (and still progressing) improvement that, by extrapolation the number of successful treatments would undoubtedly have risen to 15 if the treatment had been continued for a further 2-3 weeks. By such extrapolation the percentage of successful treatment for Invention "A" increases to 83,3%.

D) In the case of Invention "B" the trial was in fact continued for a further 14 days, following which the only remaining patient whose homocysteine level had after 42 days dropped to just above the cut-off level also qualified as a completely successful treatment, having now attained a very satisfactory level of 12,74 μ mol/l. After 56 days the success rate for Invention "B" thus amounted to a remarkable 100%. The final mean homocysteine level was 10,89 with a standard deviation of only 2.89.

E) The doubling of the dosage rate after 6 weeks produced so little effect on average plasma homocysteine levels that it can be concluded that on average the dosage rate initially selected was quite satisfactory.

F) After only 2 weeks on preparations according to the invention, average plasma homocystein levels were already 37,7% down.

Table "B" shows how plasma levels of vitamins and homocysteine changed over 8 weeks of treatment with "Invention B".

TABLE B

| Effect of vitamin supplementation on plasma levels of homocysteine, cobalamin, pyridoxal-5'-phosphate and folate | | | | | | |
|---|---|---|---|---|---|---|
| Group | Plasma Parameter | Treatment period (weeks) | | | | |
| | | 0 | 2 | 4 | 6 | 8 |
| P V | PLP (nmol/L) | 44.5(23.6) 39.9(23.0) | 43.8(19.6) *154.4(62.1) | 42.2(21.7) *157.0(72.4) | 41.3(19.4) *157.1(69.0) | 45.4(26.8) *297.3(114.3) |
| P V | cobalamin (pmol/L) | 215.7(91.3) 220.3(74.4) | 213.1(110.1) †313.2(99.0) | 227.8(123.8) †331.5(110.0) | 241.1(126.5) †335.5(111.7) | 196.3(77.5) *378.2(133.0) |
| P V | Folate (nmol/L) | 6.2(4.3) 5.6(3.3) | 5.8(3.4) †20.6(15.6) | 7.1(5.1) *27.4(13.8) | 8.3(6.6) †23.6(14.6) | 6.2(3.1) *39.3(21.9) |
| P V | Homocysteine (μmol/L) | 24.0(11.3) 28.6(15.2) | 24.0(12.8) 18.1(9.0) | 25.2(13.0) †14.1(4.7) | 22.1(8.5) *11.5(3.2) | 22.3(9.6) *10.9(3.0) |
| Group P received placebo, while group V received a vitamin supplement. Vitamin and placebo doses were doubled after 6 weeks. The p-values refer to comparisons between the groups P and V for each parameter respectively. Results are expressed as mean (SD). | | | | | | |

*p < 0.001
†p < 0.05

The following is a summary of the results of the study in the group as a whole in relation to single vitamin levels:

Average plasma homocysteine (whole group, n = 44) 26, 3nmol/l

All 3 vitamins deficient (n = 3); average plasma homocysteine: 51,5 nmol/l

Concentration of all 3 vitamins "normal" (n = 7); average plasma homocysteine 18,6 nmol/l.

Only folate deficient (n = 12); average plasma homocysteine 27,7 nmol/l

Only vitamin B12 deficient (n = 4); average plasma homocysteine 26,4 nmol/l

Only vitamin B6 deficient: none

Prevalence of vitamin deficiency in hyperhomocysteinemics (defined as plasma homocysteine >16,3 μmol/l:

-

TABLE C

| | | B6 | B12 | folate |
|---|---|---|---|---|
| Cutt-off point | | 30 μmol/l | 200 pmol/ | 5 nmol/l |
| Prevalence of deficiency % | | 25 | 56,8 | 59,1 |

## Example 2

The following is the composition of Invention "A" as used in the trials of Example 1.

Gelatine capsules, filled with a granulate, formulated for timed release (over about 8 hours) in a manner known per se, contained per capsule:-

| | |
|---|---|
| Pyridoxine | 10 mg |
| thiamine | 3 mg |
| riboflavine | 4 mg |
| nicotinamide | 20 mg |
| cyanocobalamine | 50 µg |
| ascorbic acid | 200 mg |
| folic acid | 1 mg |
| calcium panthothenate | 10 mg. |

## Example 3

Ingredients

| A. | Rapid release layer | mg per tablet |
|---|---|---|
| | Cyanocobalamin | 0,02 |
| | Beta-Carotene | 3,0 |
| | Microcrystalline cellulose | 151,3 |
| | Magnesium stearate | 0.7 |

| B. | Slow release layer | |
|---|---|---|
| | Pyridoxal (as hydrochloride) | 2,5 |
| | Pyridoxine (as hydrochloride) | 7,5 |
| | Folic acid | 0,650 |
| | Calcium hydrogen phosphate | 120,0 |
| | Acrylic resins | 8,0 |
| | Ethyl cellulose | 3,0 |
| | Povidone | 2,5 |
| | Magnesium stearate | 2,7 |

Method

A. Rapid release layer

1. Triturate the cyanocobalamin with some of the microcystalline cellulose.
2. Blend in the Beta-Carotene and the balance of the microcrystalline cellulose.
3. Lubricate the powder with magnesium stearate.

B. Slow release layer

1. Blend the ingredients and granulate with Povidone/alcohol.
2. Dry the granules in an oven.
3. Sift the dried granules and lubricate with magnesium stearate.

C. Tabletting

1. Compress the tablets in a double-layer press.

The tablets of Invention "B" in Example 1 were made in accordance with the method described above, albeit with a different ratio of active ingredients as explained in Example 1.

Dosage:    1 tablet per day, evenings after meals.
                In severe cases the dosage may be increased.

**Example 4**

Slow release betaine tablets

Tablets with sustained release properties with the following composition were preferred:-

| Per tablet | |
|---|---|
| Pyridoxal (as hydrochloride) | 2,5 mg |
| Pyridoxine (as hydrochloride) | 2,5 mg |
| Folic acid | 0,5 mg |
| Betaine hydrate | 0,5 g |
| Calcium hydrogen phosphate | 140,0 mg |
| Acrylic resins | 20,0 mg |
| Ethyl cellulose | 15,0 mg |
| Povidone | 10,0 mg |
| Magnesium stearate | 4,0 mg. |

1. Blend the ingredients and granulate with povidone/alcohol.
2. Dry the granules in an oven.
3. Silt the dried granules and lubricate with magnesium stearate.
4. Compress the tablets in the usual manner.
5. Finally, sugar coat the tablets in such a manner that each tablet contains in the sugar layer 0,25 mg of cyanocobalamin.

Dosage:    2-6 tablets daily.

This formulation is intended mainly for patients who respond inadequately to the formulations of examples 1-3.

**Example 5**

Injectable solutions

One dosage unit of injectable solution contains 1000 $\mu$g hydroxycobalamin, 1100 $\mu$g folic acid and 5,0 mg pyridoxine, dissolved in saline for intramuscular injection.

The daily dosage is 1 to 5 dosage units, preferably spread evenly over the day.

The same composition can also be administered, suitably diluted by infusion.

**Example 6**

A suitably buffered and stabilised injectable solution containing the following ingredients per dosage unit is prepared:-

| Hydroxycobalamine | 1000 $\mu$g |
|---|---|
| Folic acid | 1100 $\mu$g |
| Pyridoxal | 5 mg |

The solution can also be prepared in a vehicle which retards absorption according to methods known in the art.

The dosage and administration is as in Example 5.

**Example 7**

Dual layer tablets are prepared as in Example 3 wherein the rapid release layer contains per dosage unit 400 $\mu$g cyanocobalamin and the slow-rlease layer contains:-

| PN.HC*l* | 12,4 mg |
|---|---|
| folic acid | 1 mg |
| ascorbic acid | 500 mg |
| d-$\alpha$-tocopherol acetate | 400 mg. |

The recommended daily dosage per day/70 kg is:

**Example 8**

A further clinical trial was conducted to compare the effect of a composition according to the invention with that of the individual vitamins (B6, B12 and folate) on plasma homocysteine levels in randomised groups of patients with hyperhomocysteinemia.

100 patients with elevated plasma homocysteine (>16,3 $\mu$mol/*l*) were divided into 5 groups:-

| GROUP | SUPPLEMENT | |
|---|---|---|
| A | Placebo | |
| B | B12(0,4mg) | |
| C | Pyridoxine 8mg<br>Pyridoxal 2mg | Total<br>B6 10mg |
| D | Folate 0,65mg | |
| E | Invention (the combination B + C + D) | |

The preparation according to the invention was formulated as follows (per oral dosage unit):-

| a) | (i)<br>(ii) | pyridoxal<br>pyridoxine | 2 mg<br>8 mg; (i) + (ii) = 10 mg |
|---|---|---|---|
| b)<br>c) | | folate<br>cyanocobalamin | 0,65 mg<br>0,4 mg. |

The preparation according the invention was formulated as a multi-phase, controlled release formulation with a) and b) contained in a slow-release matrix (90% in 4-6 his) and c) in immediate release form (<30 min.). Likewise, the formulations for groups B, C and D were formulated with the contents for the individual vitamins and with release characteristics as in the formulation for group E.

The following is a summary of results at the beginning of the trial and after 3 weeks. (Results shown only of 78 patients who completed the trial.)

| RESULTS | | | | | |
|---|---|---|---|---|---|
| | Placebo (n = 16) | B12 (n = 15) | B6 (n = 17) | folate (n = 19) | Invention (n = 11) |
| P-HC**) (before) | 25,6 | 23,5 | 29,8 | 28,7 | 27,6 |
| P-HC (after) | 26 | 21,6 | 28,7 | 16,4 | 12,1 |
| %change | +1,5 | -8,1 | -3,7 | -42,3 | -56,2 |
| p | NS | NS | NS | <0,001 | <0,001 |
| %patients | 1/16 | 3/15 | 3/17 | 11/19 | 10/11 |
| normalised | 6,3% | 20% | 17,6% | 58% | 91% *) |
| 100 hyperhomocysteinemics (P-HC >16,3 $\mu$mol/$l$) | | | | | |

*) reduced 100%

**) Plasma homocysteine

As regards the results obtained in connection with the composition in accordance with the invention, it is important to note that all patients responded very favourably to the treatment and that the single patient who, after 6 weeks had not yet reached a "normal" homocysteine level, had very nearly reached that level and would probably have reached the "normal" level if the trial had been extended over a slightly longer period.

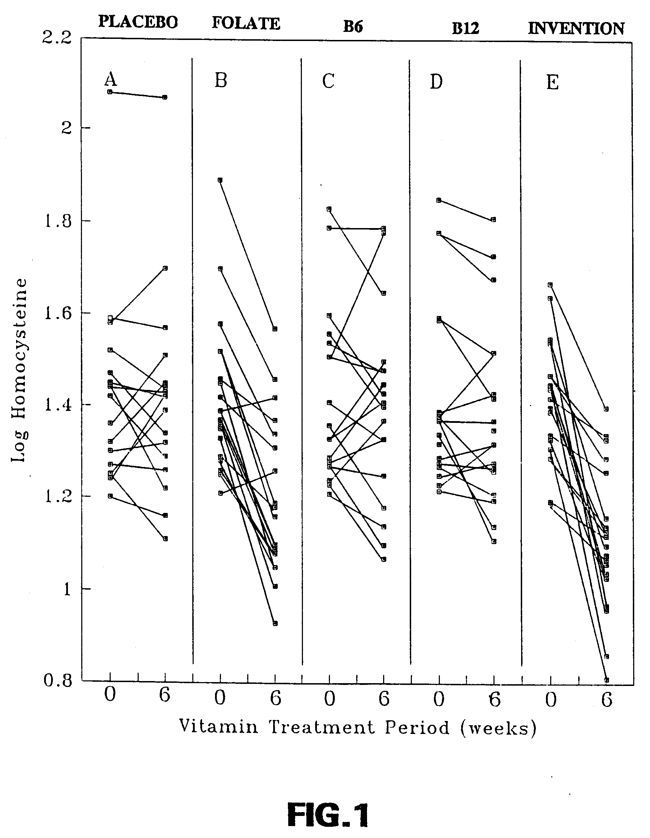

The results are also graphically represented in Fig. 1 of the drawings.

The results lead to the following conclusions:-

Neither vitamin B12 nor vitamin B6 alone achieved significant effects when taking each group as a whole. However, within each group there were about 20% that did respond.

The composition according to the invention is nearly twice as effective as folate alone. This indicates a significantly more than a purely additive effect of the three component combination (synergism).

The trial groups represented an average population age. Separate tests have already indicated that, had the average age been higher, the effect of vitamin B12 would probably have been greater. Thus one inventive aspect of this disclosure resides in the clear recognition that different age groups have different requirements for the three individual vitamins in relation to their effects on plasma homocysteine, thus again demonstrating the advantage of the combined use of the three vitamins.

Separate tests have also indicated that in patients, particularly younger patients having unusually high plasma homocysteine levels, the probability of a vitamin B6 defiency is higher than average. This provides additional support for the inclusion of B6 supplementation. Applicant has established that about 20% of an average population suffer from a genetically reduced ability to convert pyridoxine into pyridoxal and hence into intracellular pyridoxal phosphate (the only active form of vitamin B6). This genetic defiency is counteracted by supplying part of the vitamin B6 in the form of pyridoxal. Particularly in younger patients with such genetic vitamin B6 defect, the pyridoxal supplementation is more important than appears from the average results.

The tests show that (in contrast to prior art reports teaching the use of folate alone at levels 5 to 20 times higher than in the present trials), nearly 50% of patients do not respond sufficiently to folate alone and 10-20% do not respond to folate alone at all, (not even if the folate dosage rate is greatly increased). By way of contrast, the combination in accordance with the invention, using very low folate concentrations, achieved close on 100% success.

A comparison of the results of the present trial with those of the trial according to Example 1 shows that, in the combination of vitamin B6, vitamin B12 and folate it was possible to reduce the folate dosage rate significantly without loss of efficacy.

## Claims

1. The use in the manufacture of a pharmaceutical preparation for lowering levels of homocysteine or for the prophylaxis or treatment of elevated levels of homocysteine or of clinical conditions associated therewith in a patient of a combination comprising
    a) vitamin B6;
    b) folate or a suitable active metabolite of folate or a substance which releases folate in vivo;
    c) vitamin B12, with or without intrinsic factor.

**2.** A pharmaceutical preparation which comprises in combination, each in a concentration and form effective to suppress homocysteine levels in plasma

a) vitamin B6;

b) folate or a suitable active metabolite of folate or a substance which releases folate in vivo;

c) vitamin B12, with or without intrinsic factor,

and wherein, if the preparation is for oral use and any of the vitamin B6 is represented by pyridoxine (PN), such PN is formulated in slow-release form, and wherein the ingredients a) - c) are present in the following ratios by weight

a):b) from 100:1 to 1:10 and

b):c) from 100:1 to 1:50.

**3.** A use as claimed in claim 1, characterised in that, in the preparation the ingredients a) - c) are present in the following ratios by weight calculated on the basis of pure unphosphorylated pyridoxal (PL), pure vitamin B12 and pure folic acid:

a):b) from 100:1 to 1:10 and

b):c) from 100:1 to 1:50

**4.** The invention as claimed in claim 1 or 2, characterised in that the ratios are:

a):b) from 50:1 to 1:1,5

b):c) from 15:1 to 1:2

**5.** The invention as claimed in claim 1 or 2, characterised in that the ratios are:

a):b) from 20:1 to 2,5:1

b):c) from 4:1 to 1:1

and in particular:-

a):b) from 20:1 to 5:1

b):c) from 2:1 to 1:2

**6.** The invention as claimed in any one of claims 1 to 5, characterised in that the preparation is formulated to provide approximate daily dosages as follows (µg/d/kg body weight):

| | a) Vitamin B6 | b) Folic Acid | c) Vitamin B12 |
|---|---|---|---|
| Broadest range | 15-750 | 1,5-150 | 1,5-75 |
| preferred range | 30 - 400 | 7,5 - 50 | 3 - 15 |
| more preferred range | 75 - 250 | 10 - 30 | 7 - 10 |
| typical example | 150 | 15 | 7,5 |

**7.** The invention as claimed in any one of claims 1 to 6, characterised in that the preparation is formulated to make available to the patient the vitamin B6 and preferably also the folate over a period of more than 1 hour and to make available an effective dosage of the vitamin B12 in less than 1 hour after administration.

**8.** The invention as claimed in any one of claims 1 to 7, characterised in that the preparation is galenically formulated for parenteral administration, preferably by infusion or by intramuscular injection.

**9.** The invention as claimed in any one of claims 1 to 8, characterised in that the preparation combines all three essential ingredients in a single dosage form, preferably designed for oral administration.

**10.** The invention as claimed in any one of claims 1 to 8, characterised in that separate ingredients of the preparation are provided in separate distinctive dosage forms, e.g. capsules, tablets or coated tablets, preferably combined in a single package e.g. a blister pack or similarly ordered package, designed to facilitate or prescribe to the user the combined administration of the dosage units according to a specific dosage regimen.

11. The invention as claimed in claim 10, characterised in that the dosage regimen is time programmed, providing for different dosage rates during different periods of a course of treatment.

12. The invention as claimed in any one of claims 1 to 11, characterised in that at least the vitamin B6 is formulated for slow release of the compound over a period of not less than 2 hours.

13. The invention as claimed in claim 12, characterised in that the folate or precursor thereof is also formulated for slow release of the compound over a period of not less than 2 hours.

14. The invention as claimed in claim 12 or 13, characterised in that the vitamin B12 (with or without intrinsic factor) is galenically formulated for the preparation to release the vitamin B12 (with or without intrinsic factor) to the patient, more particularly the stomach in less than 1 hour after administration.

15. The invention as claimed in any one of claims 1 to 14, characterised in that the vitamin B6 is represented at least in part by pyridoxal (PL) or a compound which readily releases PL in vivo without the intervention of oxidase or oxygen, and wherein preferably PL or its precursor is provided in a non-phosphorylated form.

16. The invention as claimed in any one of claims 1 to 15, characterised in that vitamin B12 is used in the form of cyanocobalamin or hydroxycobalamin or both the the vitamin B6 being preferably represented by a mixture of PN and PL in the following ratio:-
    PL:PN =          from 1:10 to 10:1
    preferably       from 1:6 to 4:1
    more preferably  from 1:6 to 1:1
    e.g. 1:4

17. The invention as claimed in any one of claims 1 to 16, wherein the vitamin B6 is galenically formulated for at least 90% to be released over a period of 2 to 8 hours, preferably 3 to 6 hours, more preferably 3 to 4 hours and the B12 over a period of 5 - 30 minutes, whereas the vitamin B12 (with or without intrinsic factor) is formulated to be released in less than $\frac{1}{2}$ hour.

18. The invention as claimed in any one of claims 16 or 17, characterised in that the folate as well is galenically formulated to be released by the composition in not less than 2 hours, preferably 2 to 8 hours, more preferably 3 to 6 hours, e.g. 4 to 6 hours.

19. The invention as claimed in any one of claims 16 to 18, characterised in that the composition contains vitamin B6 and preferably also folate in a part of the composition adapted as a slow, timed release composition and containing the vitamin B12 (with or without intrinsic factor) in another part adapted for fast release and wherein preferably the composition is provided as:
    a) a bi-layered tablet,
    b) a coated tablet, containing the vitamin B12 in a rapidly dissolving coating; or
    c) a pharmaceutical composition in granular form, loose or in a capsule.

20. A pharmaceutical composition comprising folate and vitamin B12 in a combination, wherein the former is galenically formulated or adapted to be administered in a slow, timed release manner and the latter is formulated or adapted for fast release.

21. The invention as claimed in any one of claims 1 to 20, characterised in that the composition or preparation in addition comprises choline or betaine or both, and these are preferably formulated in slow release form, the choline and/or betaine being preferably incorporated to provide a daily dosage rate of 0,01 - 0,1 g/d/kg body weight.

22. The invention as claimed in any one of claims 1 to 21, characterised in that one or more of the active ingredients is formulated for the direct absorption of one or more of these vitamins through various tissues and membranes including the skin, nasal membranes, sub-lingual membranes, rectal membranes, e.g. formulated as
    1) sub-lingual tablets,
    2) plasters designed for skin absorption,

3) rectal pesaries,
4) suitably formulated gels or ointments, or
5) suitably formulated and concentrated solutions (aqueous, non-aqueous) of vitamins applied to the skin and/or other suitable tissues.

23. The invention as claimed in any one of claims 1 to 22, characterised in that the preparation or composition in addition comprises one or more antioxidants (e.g. $\beta$-carotene, vitamin E, vitamin C, coenzyme Q, etc.).

24. A plaster containing PL, vitamin B12 and folate in a suitable carrier for transdermal absorption.

25. A transdermal plaster containing pyridoxal as an active ingredient.

26. The invention as claimed in any one of claims 1 to 24, characterised in that betaine and/or choline wholly or in part replaces vitamin B12 or folate or both.

27. The invention as claimed in any one of claims 17 to 21, wherein vitamin B6 is an optional ingredient.

28. The invention as claimed in any one of claims 2 and 4 to 27 for lowering levels of homocysteine or for the prophylaxis or treatment of elevated levels of homocysteine.

**FIG.1**

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X,Y | EP-A-0 379 936 (VESTA MEDICINES (PROPRIETARY) LIMITED) <br> * the whole document, especially examples 14 and 15 * <br> --- | 1-28 | A61K31/68 //(A61K31/68, 31:505,31:44) |
| D,P, X | GB-A-2 254 556 (FISONS PLC) <br><br> * page 1, line 1-7 * <br> * page 4, line 8-23 * <br> * page 6, line 6-9; claims 1,4,12,13,34,42,44-46 * <br> --- | 2,4-28 | |
| D,X | GB-A-1 201 014 (E. MERCK AG) <br> * page 2, line 27-38; example 6 * <br> --- | 2,4-28 | |
| X | WO-A-91 11117 (BOARD OF REGENTS, THE UNIVERSITY OF TEXAS SYSTEM) <br> * abstract * <br> * page 23, line 29 - page 24, line 2; claims * <br> --- | 2,4-28 | |
| X | ROTE LISTE. BUNDESVERBAND DER PHARMAZEUTISCHEN INDUSTRIE E.V., EDITIO CANTOR, AULENDORF, 1992. <br> * preparations containing vitamin B6, vitamin B12, and folic acid * <br> --- | 2,4-28 | **TECHNICAL FIELDS SEARCHED** (Int.Cl.5) <br><br> A61K |
| X | DIALOG INFORMATION SERVICES, INC., FILE 446: IMSWORLD NEW PRODUCT LAUNCHES, AN=01238461 <br> * Hepabionta * <br> --- | 2,4-28 | |
| X | DIALOG INFORMATION SERVICES, INC., FILE 446: IMSWORLD NEW PRODUCT LAUNCHES, AN=01235212 <br> * Anemo Funk * <br> --- <br><br> -/-- | 2,4-28 | |

The present search report has been drawn up for all claims

| Place of search | Date of completion of the search | Examiner |
|---|---|---|
| THE HAGUE | 1 February 1994 | Orviz Diaz, P |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | DIALOG INFORMATION SERVICES, INC., FILE 446: IMSWORLD NEW PRODUCT LAUNCHES, AN=01240111 <br> * Normovite Antianem * <br> --- | 2,4-28 | |
| X | DIALOG INFORMATION SERVICES, INC., FILE 446: IMSWORLD NEW PRODUCT LAUNCHES, AN=01148023 <br> * Melicedin SRS * <br> --- | 2,4-28 | |
| D,P, X <br><br> L | AM. J. CLIN. NUTR <br> vol. 57, no. 1 , January 1993 <br> pages 47 - 53 <br> J.B. UBBINK 'Vitamin B-12, vitamin B-6, and folate nutritional status in men with hyperhomocysteinemia' <br> * the whole article, especially page 49, left column, lines 1-5* <br> (The vitamin supplement ROYL-CYNOR is mentioned) <br> --- | 1-28 | |
| P,X | CLIN. INVEST. <br> vol. 71, no. 12 , 1993 <br> pages 993 - 998 <br> J.B. UBBINK 'Hyperhomocysteinemia and the response to vitamin supplementation.' <br> * the whole document * <br> --- | 1-28 | TECHNICAL FIELDS SEARCHED    (Int.Cl.5) |
| X | ATHEROSCLEROSIS <br> vol. 75, no. 1 , 1989 <br> pages 1 - 6 <br> A.J. OLSZEWSKI 'Reduction of plasma lipid and homocysteine levels by pyridoxine, folate, cobalamin, choline, riboflavin, and troxerutin in atherosclerosis.' <br> * the whole document * <br> --- <br> -/-- | 1-28 | |

The present search report has been drawn up for all claims

| Place of search | Date of completion of the search | Examiner |
|---|---|---|
| THE HAGUE | 1 February 1994 | Orviz Diaz, P |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| L | ATHEROSCLEROSIS vol. 88, no. 1 , 1991 pages 97 - 98 A.J. OLSZEWSKI 'Homocysteine content of plasma in ischemic heart disease, the reducing effect of pyridoxine, folate, cobalamin, choline, riboflavin, and troxerutin. Correction of a calculation error.' * the whole document * (Correction of the results described in the previous reference) --- | 1-28 | |
| D,Y | SCAND. J. CLIN. LAB. INVEST. vol. 48, no. 3 , 1988 pages 215 - 221 L.E. BRATTSTRÖM 'Folic acid-an inocuous means to reduce plasma homocysteine.' * the whole document * --- | 1-28 | |
| Y | ATHEROSCLEROSIS vol. 81, no. 1 , 1990 pages 51 - 60 L. BRATTSTRÖM 'Impaired homocysteine metabolism in early-onset cerebral and peripheral occlusive arterial disease.' * the whole document * --- | 1-28 | TECHNICAL FIELDS SEARCHED (Int.Cl.5) |
| Y | TRENDS PHARMACOL. SCI. vol. 11, no. 10 , 1990 pages 411 - 416 H. REFSUM 'Clinical significance of pharmacological modulation of homocysteine metabolism.' * the whole document * --- | 1-28 | |

-/--

The present search report has been drawn up for all claims

| Place of search | Date of completion of the search | Examiner |
|---|---|---|
| THE HAGUE | 1 February 1994 | Orviz Diaz, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | EP-A-0 270 026 (VESTA MEDICINES (PROPRIETARY) LIMITED) <br> * claims * <br> ----- | 1-28 | |

TECHNICAL FIELDS SEARCHED (Int.Cl.5)

The present search report has been drawn up for all claims

| Place of search | Date of completion of the search | Examiner |
|---|---|---|
| THE HAGUE | 1 February 1994 | Orviz Diaz, P |

EPO FORM 1503 03.82 (P04C01)